# EUROPÄISCHE PATENTSCHRIFT

(11) **EP 1 833 758 B1**
(45) Veröffentlichungstag und Bekanntmachung des Hinweises auf die Patenterteilung: **26.08.2009**
(21) Anmeldenummer: 05795058.6
(22) Anmeldetag: 20.10.2005
(51) Int. Cl.: C01B 7/07, C01B 7/09, B01D 53/14, C01B 7/19, C07C 51/64, C07D 233/54

(54) **VERFAHREN ZUR GASTRENNUNG**
METHOD FOR SEPARATING GAS
PROCEDE POUR SEPARER DU GAZ

(30) Priorität: 27.10.2004 EP 04025509; 22.12.2004 DE 102004061780
(43) Veröffentlichungstag der Anmeldung: 19.09.2007
(73) Patentinhaber: Solvay Fluor GmbH, 30173 Hannover (DE)
(72) Erfinder: OLSCHIMKE, Jens, 30519 Hannover (DE); BRAUKMÜLLER, Saskia, 31157 Sarstedt (DE); BROSCH, Carsten, 30926 Seelze (DE)
(74) Vertreter: Jacques, Philippe
(86) Internationale Anmeldenummer: PCT/EP2005/011267
(87) Internationale Veröffentlichungsnummer: WO 2006/045518

(56) Entgegenhaltungen:
- EP-A- 1 394 109
- WO-A-02/074718
- US-A1- 2002 189 444
- US-A1- 2003 204 041

## Beschreibung

Die Erfindung bezieht sich auf ein Verfahren zur Trennung von Gemischen, die HCl, HF und/oder HBr und andere Bestandteile enthalten, insbesondere solche Gemische, die C(O)F₂, Phosphorpentafluorid oder bestimmte Säu refluoride und HCl sowie gegebenenfalls HF enthalten.

Organische gasförmige Säurefluoride wie CF₃C(O)F oder CHF₂C(O)F sind Ausgangsmaterialien für fluorhaltige organische Verbindungen, beispielsweise für Fluorvinylether, die ihrerseits Comonomere für Harze oder Elastomere darstellen. CH₃C(O)F ist als Schädlingsbekämpfungsmittel vorgeschlagen worden. Säurefluoride können herstellungsbedingt HCl oder HBr enthalten, manchmal zusätzlich auch HF.

Phosphorpentafluorid ist Zwischenprodukt bei der Herstellung von Elektrolytsalzen für Lithiumionenbatterien. Es kann beispielsweise mit Lithiumfluorid zu Lithiumhexafluorphosphat umgesetzt werden. Das Phosphorpentafluorid kann aus Phosphorpentachlorid oder Phosphortrichlorid und Chlor sowie Fluorwasserstoff hergestellt werden. Bei dieser Reaktion fällt HCl an, die vom Phosphorpentafluorid abgetrennt werden muss.

Carbonylfluorid ist als neues Ätzgas in der Halbleitertechnik und für die Reinigung von CVD-Reaktoren vorgeschlagen worden. Die Europäische Patentanmeldung 04005421.5 (entspricht der WO05/085129) beschreibt ein photochemisches Verfahren zur Herstellung von C(O)F₂ aus CHClF₂. Das dort beschriebene Verfahren sieht die Herstellung von C(O)F₂ durch Photooxidation von CHClF₂ mit Sauerstoff vor. Dabei wird Licht eingestrahlt, das nicht aus einer einzigen Wellenlänge besteht, sondern einen Spektralbereich aufweist, der mindestens 50 nm umfasst (d. h. der Lichtanteil mit niedrigster Wellenlänge und der Lichtanteil mit der höchsten Wellenlänge liegen mindestens 50 nm auseinander).

Der Druck im Reaktor entspricht bei dem dort beschriebenen Verfahren bevorzugt mindestens dem Umgebungsdruck, also 1 bar (abs.). Er kann auch darüber liegen. Der Druck liegt bevorzugt im Bereich von 1 bar (abs.) bis 11 bar (abs.). Die Temperatur liegt bevorzugt im Bereich von 20 bis 300 °C, besonders im Bereich von 30 bis 300 °C und insbesondere im Bereich von 30 bis 90 °C. Vorteilhaft wählt man die Bedingungen hi n-sichtlich Druck und Temperatur derart, dass das Reaktionsgemisch gasförmig bleibt.

Ganz besonders bevorzugt arbeitet man bei dem dort beschriebenen Verfahren drucklos. Der Begriff "drucklos" bedeutet dort, dass auf die Reaktionsmischung außer dem Umgebungsdruck (d.h. etwa 1 bar), dem Förderdruck des Sauerstoffgases (bzw. des sauerstoffhaltigen Gases, man kann z. B. Luft oder Sauerstoff/Inertgas-Gemische einsetzen) und des gegebenenfalls eingesetzten Chlors sowie dem sich gegebenenfalls ausbildenden Druck durch bei der Reaktion entstehendes Chlorwasserstoffgas kein zusätzlicher Druck einwirkt. Der Gesamtdruck im Reaktor ist dann zweckmäßig kleiner als 2 bar absolut, je nach Förderdruck sogar kleiner als 1,5 bar absolut, aber größer als der Umgebungsdruck.

Das Verfahren kann batchweise oder bevorzugt kontinuierlich durchgeführt werden. Vorzugsweise geht man so vor, dass man kontinuierlich Ausgangsmaterial (das entsprechende Edukt, Sauerstoff bzw. ein Sauerstoff enthaltendes Gas wie Luft oder reinen Sauerstoff und gegebenenfalls Chlor) in eine Durchflussapparatur einspeist und entsprechend der eingespeisten Menge kontinuierlich Reaktionsprodukt abzieht. Die durchschnittliche Verweilzeit im Reaktionsgefäß liegt vorteilhaft zwischen 0,01 und 30 Minuten, bevorzugt zwischen 0,1 bis 3 min, besonders bevorzugt zwischen 0,3 und 1,5 Minuten. Die optimale durchschnittliche Verweilzeit, die u.a. von der Art der Larn pen, der Strahlungsleistung der Lampen und von geometrischen Parametern der Bestrahlungsapparatur abhängig ist, kann man durch einfache Handversuche und Analyse des Produktstroms, beispielsweise durch Gaschromatographie, ermitteln. Vorteilhaft kann es auch sein, die Reaktionsmischung beispielsweise durch geeignete Einbauten im Reaktor gut zu verwirbeln. Die optimale Verweilzeit bei batchweiser Durchführung kann in gleicher Weise ermittelt werden.

Eine Ausführungsform sieht die Photooxidation in Abwesenheit von Chlor oder anderen Radikalinitiatoren oder Aktivatoren vor. Beispielsweise kann die Bestrahlung durch Quarzglas hindurch vorgenommen werden; andere Bauteile des Reaktors, die nicht zwischen Lichtquelle und Reaktionsgemisch angeordnet sind, können natürlich aus beliebigen Bauteilen, z. B. auch aus Borsilikatglas sein, welches bestimmte Strahlungsanteile filtert (siehe unten). Als Strahler eigenen sich übliche Strahler, die beispielsweise Strahlung im Bereich von 250 bis 400 nm oder sogar bis 600 nm abgeben (das Spektrum kann auch über die untere oder obere Grenze hinausgehen).

Eine weitere, besonders bevorzugte Ausführungsform in jener Europäischen Patentanmeldung sieht die Bestrahlung in Anwesenheit von elementarem Chlor unter Bestrahlung mit Licht einer Wellenlänge von ≥ 280 nm vor, wobei pro Gewichtsteil CHClF₂ maximal 0,6 Gewichtsteile elementares Chlor im Reaktionsgemisch enthalten sind. Bevorzugt werden pro Mol CHClF₂ 1 bis 50 mol-% Chlor, vorzugsweise 5 bis 20 mol-% elementares Chlor eingesetzt.

Umsatzrate, Ausbeute und Selektivität sind bei dem dort beschriebenen Verfah ren besonders hoch, wenn man in Anwesenheit von elementarem Chlor umsetzt und eine aktivierende Bestrahlung mit Licht einer Wellenlänge λ ≥ 280 nm vornimmt. Frequenzen einer Wellenlänge unterhalb von 280 nm sind dann im Wesentlichen aus dem Frequenzspektrum ausgeblendet. Dies kann man dadurch bewirken, dass man Bestrahlungslampen verwendet, die nur Licht einer Wellenlänge oberhalb oder bei 280 nm abstrahlen, und/oder man verwendet Mittel, die die entsprechenden Frequenzen aus dem abgestrahlten Licht ausblenden. Beispielsweise kann man durch Glas bestrahlen, welches nur für Licht einer Wellenlänge von 280 nm oder darüber durchlässig ist, also den Strahlungsanteil mit kürzeren Wellenlängen herausfiltert. Gut geeignet dafür sind beispielsweise Borosilikatgläser. Geeignete Gläser enthalten beispielsweise 7 bis 13 % B₂O₃, 70 bis 80 % SiO₂, ferner 2 bis 7 % Al₂O₃ und 4 bis 8 % Na₂O + K₂O sowie 0 bis 5% Erdalkalimetalloxide (jeweils Gew.-%). Bekannte Marken für Borosilikatgläser sind Duran, Pyrex und Solidex.

Das Molverhältnis zwischen dem Edukt und Sauerstoff kann bei dem in der genannten Europäischen Patentanmeldung beschriebenen Verfahren in einem weiten Bereich schwanken, zweckmäßig setzt man jedoch mindestens 0,4 Mol Sauerstoff pro Mol Ausgangsverbindung ein. Besonders gute Ergebnisse werden erzielt, wenn das Molverhältnis zwischen der Ausgangsverbindung und dem Sauerstoff im Bereich von 1:0,4 bis 1:1, insbesondere 1 :0,4 bis 1:0,6 liegt. Der Sauerstoff kann wie gesagt in Form von Luft eingesetzt werden. Bevorzugt setzt man den Sauerstoff in Form eines O₂/Inertgas-Gemisches, insbesondere aber als reinen Sauerstoff ein. In Bezug auf die Produktreinheit ist es wünschenswert, dass möglichst wenig Wasser bei der Umsetzung vorhanden ist (beispielsweise weniger als 30 ppm). Gewünschtenfalls kann man die Reaktanten in bekannter Weise von mitgeschlepptem Wasser befreien, z. B. mittels Molekularsieb. Die Trennung von C(O)F₂ und HCl ist beispielsweise durch Druckdestillation möglich.

Aufgrund der eng beieinander liegenden Siedepunkte von C(O)F₂ und HCl ist eine destillative Trennung allerdings aufwendig.

Gemische, die HF enthalten, können beispielsweise Resultat einer Fluorierungsreaktion mit Fluorwasserstoff sein. So kann man Carbonsäurefluoride durch die Umsetzung von Carbonsäurechloriden und HF herstellen oder photochemisch (US-A 6489510). Diese Gemische enthalten gewöhnlich auch HCl.

Über die vorstehend beschriebenen Trennprobleme HCl/Carbonsäurefluorid, HF/Carbonsäurefluorid, HF/HCl/Carbonsäurefluorid, HCl/PF₅ bzw. HCl/C(O)F₂ hinaus kann es generell wünschenswert sein, HF, HCl oder auch HBr aus Gasgemischen, die HF, HCl bzw. HBr zusammen mit anderen Bestandteilen enthalten, abzutrennen bzw. diese Gasgemische aufzutrennen, so dass man Gasgemische oder reine Gase erhält, die an HF, HCl bzw. HBr abgereichert sind bzw. in welchen die andere Komponente oder die anderen Komponenten angereichert sind.

Die Europäische Patentanmeldung EP-A 1 394 109 beschreibt ein Verfahren zur Abtrennung von HF und ähnlichen Säuren aus Säurefluoriden. Als entsäuerndes Mittel wird eine heteroaromatische Verbindung empfohlen, die einen Siedepunkt von mindestens 50 °C und als Heteroatom oder Heteroatome Stickstoff aufweist. Wie der Beschreibung der genannten Patentanmeldung zu entnehmen ist, handelt es sich dabei um Amine (diese weisen einen merklichen Dampfdruck bei Temperaturen schon unterhalb 100 °C auf) wie Imidazol oder Pyridin oder entsprechende Ionenaustauscherharze mit Aminogruppen wie der Pyridin- oder Imidazolgruppe. Als Beispiel für solche Polymere werden Polyvinyl-(4-pyridin) und Polyvinyl-(2-pyridin) genannt. Ionische Flüssigkeiten werden nicht eingesetzt.

Die internationale Patentanmeldung WO 02/074718 offenbart ionische Flüssigkeiten als selektive Zusatzstoffe für die Trennung engsiedender oder azeotroper Gemische. Bei diesen Gemischen handelt es sich um Flüssigkeiten oder kondensierte Gase, d. h. es geht um eine Flüssig-Flüssig-Trennung. Als zu trennende Gemische werden Gemische von Wasser mit Aminen, Tetrahydrofuran, Ameisensäure, Alkoholen, Acetaten, Acrylaten, Essigsäure, Gemische von Aceton und Methanol oder engsiedende Gemische wie C4-, C3-Kohlenstoffe oder Alkane/Alkene genannt. Es handelt sich also um rein organische Gemische, oftmals von Verbindungen mit mindestens 3 C-Atomen, oder um Gemische, die Wasser enthalten.

Die US-Patentanmeldung 2004/0035293 offenbart ionische Flüssigkeiten, die eine Gruppe besitzen, die Brönstedt-saure Eigenschaften aufweist, z. B. eine Sulfonsäuregruppe. Derartige ionische Flüssigkeiten können zur Gasabtrennung verwendet werden, z. B. zur Abtrennung oder Überführung von CO₂ oder COS, zur Abtrennung von Alkenen, Alkynen oder CO oder zur Katalyse.

Die US-Patentanmeldung 2002/0189444 (= US-Patent 6579343) offenbart ein Verfahren zur Gasreinigung mittels ionischer Flüssigkeiten. Beispielsweise können Wasser, CO₂, Sauerstoff und dergleichen aus Gasmischungen abgetrennt werden. Auf diese Weise können Erdgas, Luft oder Stickstoff gereinigt werden.

Aufgabe der vorl iegenden Erfindung ist es demnach, ein einfach durchzuführendes Verfahren anzugeben, mit welchem sich HF, HCl oder HBr aus Gasgemischen abtrennen lässt, so dass ei n Gasgemisch erhalten wird, welches an HF, HCl oder HBr abgereichert ist, bzw. worin die andere Komponente oder die anderen Komponenten angereichert sind.

Eine bevorzugte Aufgabe der vorliegenden Erfindung ist es, ein Verfahren anzugeben, mit welchem ein an HCl abgereichertes C(O)F₂, Phosphorpentafluorid oder Carbonsäurefluorid aus Gemischen erhalten werden kann, die C(O)F₂ bzw. Phosphorpentafluorid und HCl enthalten, bzw. mit welchem das C(O)F₂ oder Phosphorpentafluorid aus diesen Gemischen angereichert werden kann. Diese Aufgabe wird durch das Verfahren der vorliegenden Erfindung gelöst.

Im weitesten Sinne wird die vorliegende Aufgabe gelöst durch das erfindungsgemäße Verfahren zur Abtrennung von HF, HCl oder HBr aus Gasgemischen, die HF, HCl oder HBr und ein oder mehrere andere gasförmige Bestandteile enthalten, unter Kontaktieren dieser Gasgemische mit einer oder mehreren ionischen Flüssigkeiten, die HF, HCl bzw. HBr oder mindestens einen der anderen Bestandteile des Gasgemisches unterschiedlich stark sorbieren. Dabei wird keinesfalls Wasser zugesetzt, und bevorzugt ist die ionische Flüssigkeit im Wesentlichen wasserfrei (z. B. mit weniger als 0.1 Gew.-%), so dass eine Hydrolyse der Fluoride, wenn überhaupt, nur in geringem Umfang erfolgen kann. Ionische Flüssigkeiten werden im Verfahren der vorliegenden Erfindung von vornherein zugesetzt. In den zu behandelnden Gasgemischen sind die als andere Komponenten Verbindungen enthalten, die bei Normalbedingungen (25 °C, 1 bar abs.) Gase sind, wie Trifluoroacetylfluorid, Trifluoroacetylfluorid, PF₅, C(O)F₂ etc.

In der ionischen Flüssigkeit enthaltenes Wasser wird beim Durchleiten des aufzutrennenden Gasgemisches durch Reaktion mit den hydrolyseempfindlichen Bestandteilen des Gasgemisches (Carbonylfluorid, Säurefluorid bzw. PF₅) verbraucht. Die entstehenden Komponenten sind meist leichtflüchtig und leicht aus der ionischen Flüssigkeit zu entfernen.

Das Verfahren ist besonders gut zur Abtrennung von HCl geeignet und wird diesbezüglich weiter erläutert.

Das Verfahren eignet sich beispielsweise für Gasgemische, die HCl und bei Normalbedingungen gasförmige Carbonsäurechloride wie Trifluoracetylchlorid (TFAC) oder Difluoracetylchlorid (DFAC) enthalten. Es kann auch angewendet werden, um HCl aus bei Normalbedingungen gasförmigen Carbonsäurefluoriden abzutrennen, insbesondere aus Difluoracetylfluorid, Trifluoracetylfluorid, C₂F₅C(O)F oder CH₃C(O)F. Es ist auch anwendbar auf Gasgemische, die HBr und andere Bestandteile enthalten. Bevorzugt wendet man es zur Reinigung von Carbonylfluorid oder Carbonsäurechloriden bzw. Carbonsäurefluoriden mit maximal 2 C-Atomen an. Sofern HF enthalten, wird dies ebenfalls abgetrennt.

Während manche Verfahren nach dem Stand der Technik eine Trennung von flüssigen Komponenten oder kondensierten Gasen vorsehen, wird im Verfahren der vorliegenden Erfindung bevorzugt ein Gasgemisch aufgetrennt, das gasförmig mit den ionischen Flüssigkeiten kontaktiert wird, das also nicht in kondensiertem Zustand mit der ionischen Flüssigkeit in Kontakt gebracht wird.

Die oben genannte besondere Aufgabe zur Behandlung von C(O)F₂ und HCl enthaltenden Gasgemischen, zur Behandlung von Carbonsäurefluorid und HF und/oder HCl enthaltenden Gasgemischen bzw. zur Behandlung von Phosphorpentafluorid und HCl enthaltenden Gasgemischen wird durch das erfindungsgemäße Verfahren zur Gewinnung von C(O)F₂, Carbonsäurefluorid bzw. Phosphorpentafluorid, das an HF oder HCl oder HF und HCl abgereichert ist, bzw. an C(O)F₂, Carbonsäurefluorid oder Phosphorpentafluorid angereichert ist, gelöst. Dieses bevorzugte Verfahren sieht vor, dass man diese Gemische mit einer ionischen Flüssigkeit kontaktiert, die C(O)F₂, Carbonsäurefluorid bzw. Phosphorpentafluorid einerseits und HF, HCl bzw. HBr andererseits unterschiedlich stark sorbieren. Zwar ist es möglich, solche ionischen Flüssigkeiten auszuwählen, die HCl, HF bzw. HBr passieren lassen und die anderen Bestandteile sorbieren. Üblicherweise wird jedoch HCl, HF bzw. HBr von ionischen Flüssigkeiten stärker sorbiert, und ein an HCl, HF und/oder HBr abgereichertes C(O)F₂, Carbonsäurefluorid oder Phosphorpentafluo rid passiert die ionische Flüssigkeit und kann isoliert werden, bzw. das erhaltene Gas oder Gasgemisch ist an C(O)F₂, Carbonsäurefluorid oder Phosphorpentafluorid angereichert. Anhand der bevorzugten Anwendung auf Gemische, die HCl und C(O)F₂ enthalten , wird die Erfindung weiter erläutert.

Unter ionischen Flüssigkeiten werden solche verstanden, wie sie von Wasserscheid und Keim in der Angewandten Chemie 2000,112, 3926-3945 definiert sind. Ionische Flüssigkeiten sind beispielsweise als Lösungsmittel verwendbar. Wie in der genannten Druckschrift aufgeführt handelt es sich bei ionischen Flüssigkeiten um bei relativ niedrigen Temperaturen schmelzende Salze mit nichtmolekularem, ionischen Charakter. Sie sind bereits bei relativ niedrigen Temperaturen, z. B. <100 °C, flüssig und dabei relativ niedrigviskos. Sie besitzen sehr gute Löslichkeiten für eine große Anzahl organischer, anorganischer und polymerer Substanzen.

Darüber hinaus sind ionische Flüssigkeiten in der Regel nicht brennbar, nicht korrosiv und wenig viskos und zeichnen sich durch einen nicht messbaren Dampfdruck aus.

Die Ionen der in der vorliegenden Erfindung einsetzbaren ionischen Flüssigkeiten können eine oder mehrere positive bzw. negative Ladungen aufweisen, wobei Ionen mit jeweils einer positiven und einer negativen Ladung bevorzugt sind.

Ionische Flüssigkeiten, die sich zur Trennung von Substanzgemischen eignen, sind in der WO 02/074718 beschrieben. Sie basieren auf ionischen Flüssigkeiten, die Ammonium-, Guanidinium- oder Phosphoniumionen in den Kationen aufweisen. Generell wählt man die ionischen Flüssigkeiten so aus, dass sie keine chemische, zur Zersetzung führende Reaktion mit einer Komponente des aufzutrennenden Gasgemisches eingeht. Dies kann durch einfache Handversuche leicht sichergestellt werden. Sofern Bestandteile im Gasgemisch enthalten sind, die gegen Feuchtigkeit empfindlich sind, ist es zweckmäßig, Feuchtigkeit weitgehend auszuschließen, z. B. durch Trockenmittel im Reaktor, durch Spülen mit trockenem Inertgas oder ähnlichem. Das Gleiche gilt für eine etwaige Empfindlichkeit gegen Sauerstoff. Brauchbar sind auch weitere ionische Flüssigkeiten, z. B. mit Guanidiniumkationen.

Im Rahmen der vorl iegenden Erfindung werden Kationen bevorzugt, die Stickstoff enthalten.

Im folgenden werden geeignete Kationen und Anionen weiter erläutert; dabei ist für den Fachmann klar, dass die jeweilige Kationen-/Anionenpaarung ein Produkt ergeben muss, das bei einer Temperatur von nicht mehr als 100 °C flüssig sein muss, um eine nützliche Ionische Flüssigkeit zu ergeben. Besonders Vorteilhaft sind ionische Flüssigkeiten oder Gemische von ionischen Flüssigkeiten, welche bei Umgebungstemperatur (etwa 20 °C) flüssig sind.

Phosphor enthaltende Kationen, insbesondere Phosphonium-Kationen mit vier gleichen oder unterschiedlichen Alkylgruppen, wie dem Butyl-, Octyl- oder Phenylrest, sind in der vorstehend erwähnten Publikation von Wasserscheid und Keim erwähnt.

Bevorzugt sind Stickstoff enthaltende Kationen; anhand dieser Ausführungsform wird die Erfindung weiter erläutert.

Im Prinzip können alle bekannten Kationen, die mindestens einen organischen Substituenten am Ammoniumkation aufweisen, verwendet werden. Allgemein handelt es sich um primäre, sekundäre, tertiäre und quartäre Ammoniumkationen. Die Substituenten können beispielsweise lineare oder verzweigte Alkylgruppen sein, beispielsweise mit 1 bis 12 C-Atomen. Es können gleiche oder unterschiedliche Alkylsubstituenten am Stickstoffatom vorliegen. Bei den Substituenten kann es sich auch um aromatische Reste handeln beispielsweise um den Phenylrest, der gewünschtenfalls auch ein- oder mehrfach substituiert sein kann, beispielsweise durch eine oder mehrere C1-C3-Gruppen. Auch Arylalkylsubstituenten sind möglich, zum Beispiel der Benzylrest. Auch Guanidiniumkationen und Isouroniumkationen sind geeignete Kationen (derartige Verbindungen sind von Merck Darmstadt erhältlich). Die Substituenten an den Stickstoff können Wasserstoff, lineare oder verzweigte Alkylgruppen, sowie Arylgruppen sein. Die Substituenten an den Stickstoff-, Sauerstoff- und Schwefelatomen können lineare oder verzweigte Alkylgruppen, sowie Arylgruppen sein. Am Stickstoff kann auch Wasserstoff als Substituent vorhanden sein.

Auch cyclische gesättigte Ammoniumkationen sind brauchbar, beispielsweise die in der deutschen Offenlegungsschrift DE 101 14 565 genannten, gegebenenfalls substituierten mono- oder bicyclischen gesättigten Ammoniumkationen, beispielsweise Piperidinium oder durch Hydroxygruppen substituiertes Piperidinium. Auch die in dieser Offenlegungsschrift genannten Kationen von bicyclischen Aminen, insbesondere solche von 1,5-Diazabicyclo[4.3.0]non-5-en und 1,8-Diazabicyclo[5.4.0]-undec-7-en, sowie auch die durch Aminogruppen substituierten cyclischen Am ine wie Dialkylaminopiperidin und Dialkylaminopiperazin (Alkyl bedeutet C1 bis C4) sind in Form der Kationen verwendbar.

Geeignete Kationen sind auch heterocyclische Verbindungen, die mindestens ein Stickstoffatom sowie gegebenenfalls ein Sauerstoff- oder Schwefelatom enthalten und in der genannten WO 02/074718 auf den Seiten 4 bis 6 erwähnt werden. Dabei handelt es sich um Kationen auf Basis der Struktur von Pyridin, Pyridazin, Pyrimidin, Pyrazin, Imidazol, 1 H-Pyrazol, 3H-Pyrazol, 4H-Pyrazol, 1-Pyrazolin, 2-Pyrazolin, 3-Pyrazolin, 1-Imidazolin, 2-Imidazolin, 4-Imidazolin, Thiazol, Oxazol, 1,2,4-Triazol (positive Ladung am 2- bzw. 4-Stickstoffatom), 1,2,3-Triazol (positive Ladung am 2- bzw. 3-Stickstoffatom) und Pyrrolidin. Erläuterungen zu den Substituenten finden sich in der WO 02/074718 auf den Seiten 6 bis 13. Kationen von N-Alkylisochinolin, Alkyltriazolium, N-Alkylimidazolin sind ebenfalls brauchbar. Diese Strukturen können durch Wasserstoff substituiert sein. Ein oder mehrere Wasserstoffatome können auch substituiert sein, beispielsweise durch Alkylgruppen mit 1 bis 18 C-Atomen (C2-C18-Alkylgruppen können auch ein oder mehrere Sauerstoff- oder Schwefelatome oder Iminogruppen in der Kette enthalten), durch C6- bis C12-Aryl, C5-C12-Cycloalkyl oder einen fünf- bis sechsgliedrigen, Sauerstoff-, Stickstoff- oder Schwefelatome aufweisenden heterocyclischen Rest. Zwei der Substituenten können auch einen ungesättigten oder gesättigten oder aromatischen, gegebenenfalls durch ein oder mehrere Sauerstoff- oder Schwefelatome oder Iminogruppen unterbrochenen Ring bilden. Die genannten Substituenten können ihrerseits durch funktionelle Gruppen, Aryl, Alkyl, Aryloxy, Alkyloxy, Halogen, Heteroatome und/oder Heterocyclen substituiert sein.

Ein Substituent am Stickstoffatom, das die positive Ladung trägt, kann beispielsweise auch C1-C18-Alkylcarbonyl, C1-C18-Alkyloxycarbonyl, C5-C12-Cycloalkylcarbonyl oder C6-C12-Arylcarbonyl sein; dabei können auch diese Substituenten wieder durch funktionelle Gruppen, Aryl, Alkyl, Aryloxy, Alkyloxy, Halogen, Heteroatome und/oder Heterocyclen substituiert sein.

Kationen solcher Heterocyclen mit fünfgliedrigen oder sechsgliedrigen Ringen sind im Verfahren der vorliegenden Erfindung bevorzugt.

Besonders gut geeignet sind Imidazolium-, Imidazolinium-, Pyrazolium-, Oxatriazolium-, Thiatriazolium-, Pyridinium-, Pyradizinium-, Pyrimidinium- oder Pyrazinium-Kationen. Dabei können die Kohlenstoffatome bevorzugt durch Wasserstoff, C1- bis C12-Alkyl oder durch eine Hydroxy- oder CN-Gruppe substituiertes C2-C12-Alkyl sein. Das Stickstoffatom mit der positiven Ladung ist bevorzugt durch Acetyl, Methyl, Ethyl, Propyl oder n-Butyl substituiert. Es kann gegebenenfalls, wie etwaig im Ring vorhandene weitere Stickstoffatome, auch noch durch Wasserstoff oder C1-C12-Alkylgruppen substituiert sein. Bevorzugtes C1-C12-Alkyl ist Methyl, Ethyl, Propyl und n-Butyl.

Einsetzbar sind auch Oligo- und Polymere, die die vorstehend beschriebenen Kationen enthalten (siehe z.B. M. Yoshizawa, W. Ogihara und H. Ohno, Polym. Adv. Technol. 13, 589-594, 2002). Bei der vorliegenden Erfindung sind aber monomere Kationen bevorzugt.

Ganz besonders bevorzugte Kationen sind Imidazoliumkationen, die durch ein, zwei oder drei Substituenten mit je 1-24 Kohlenstoffatomen substituiert sind; dabei können die Substituenten ihrerseits beispielsweise durch Arylgruppen substituiert sein. Insbesondere bevorzugt sind 1,3-Dimethyl-imidazolium, 1-Ethyl-3-methyl-imidazolium, 1-Propyl-3-methyl-imidazolium, 1-Butyl-3-methyl-imidazolium, 1-Pentyl-3-methyl-imidazolium, 1-Hexyl-3-methyl-imidazolium, 1-Heptyl-3-methyl-imidazolium, 1-Octyl-3-methylimidazolium, 1-Nonyl-3-methyl-imidazolium, 1-Decyl-3-methyl-imidazolium, 1-Undecyl-3-methyl-imidazolium, 1-Dodecyl-3-methyl-imidazolium, 1-Benzyl-3-methyl-imidazolium, 1-Butyl-2,3-dimethyl-imidazolium. Sehr gut geeignet sind ionische Flüssigkeiten mit 1,3-Dimethyl-imidazolium, 1-Ethyl-3-methyl-imidazolium ("EMIM"), 1-Propyl-3-methylimidazolium und 1-n-Butyl-3-methyl-imidazolium ("BMIM") als Kation.

Als Anionen sind besonders solche Anionen geeignet, die zur Ausbildung von Wasserstoffbrückenbindungen geeignet sind. Stark koordinierende Anionen wie z. B. Alkylsulfate oder Arylsulfate sind besser geeignet als schwach koordinierende Anionen wie Trifluormethansulfonat und besonders Hexafluorophosphat oder Tetrafluorobonat, weil sie oft bereits bei einer einstufigen Durchführung gute Reinigungsergebnisse bewirken. Geeignet als Anionen sind auch Halogenide, besonders aber die Anionen von ein- oder mehrbasigen Sauerstoffsäuren oder deren Derivate wie Ester oder Amide, z. B. Sulfonate oder Sulfonamide. Gut geeignet sind ionische Flüssigkeiten mit folgenden Anionen: Alkylcarboxylate mit insgesamt 2 bis 8 C-Atomen, beispielsweise Acetat; durch Halogen, insbesondere durch Fluor substituierte Alkylcarboxylate, z. B. Trifluoracetat; Sulfat; Hydrogensulfat; Phosphat; Hydrogenphosphat; Dihydrogenphosphat; Alkylsulfat mit einem C1-C12-Alkylrest, der linear oder verzweigt sein kann, konkrete Anionen sind z. B. Methyl-, Ethyl-, n-Propyl-, n-Butylsulfat, bis hin zum n-Octylsulfat; Alkyl- und Dialkyl phosphat mit einem oder zwei C1-C12-Alkylresten, z.B. Methyl-, Dimethyl-, Ethyl-, Diethyl-, n-Propyl-, di-n-Propyl-, n-Butyl-, di-n-Butylphosphat;C1-C12-Alkylsulfonat, bevorzugt Cl-C4-Alkylsulfonat, z.B. Methyl-, Ethyl-, n-Propyl-, n-Butylsulfat; Sulfonat mit einer durch ein oder mehrere Halogenatome, vorzugsweise durch Fluor substituierten C1-C12-Alkylgruppe, z. B. Trifluormethylsulfonat (Triflat); Arylsulfonat, z. B. Tosylat; Phosphonat mit einer C1-C12-Alkylgruppe, die direkt am Phosphor gebunden ist, z.B. Methyl-, Ethyl-, n-Propyl-, n-Butylphosphonat; Phosphonat mit einer C1-C12-Alkylgruppe, die durch ein oder mehrere Halogenatome, vorzugsweise Fluor, substituiert und direkt am Phosphor gebunden ist, z. B. Trifluormethylphosphonat; Ester der genannten Phosphonate mit einer C1-C12-Alkylgruppe, die gewünschtenfalls auch durch ein oder mehrere Hatogenatome, vorzugsweise Fluoratome, substituiert sein kann; Imide von Bis(C1-C12-Alkyl)sulfonat, wobei die Alkylgruppen gewünschtenfalls durch ein oder mehrere Halogenatome, vorzugsweise Fluor, substituiert sein können, z. B. Bis(Trifluormethyl-sulfonyl)imid.

Bevorzugte Anionen sind C1-C12-Alkylsulfate, besonders C1-C4-Alkylsulfate, insbesondere Methyl- und Ethylsulfat, sowie, wie weiter unten noch erläutert wird, Triflat und Tosylat sowie deren Gemische.

Ohne dass eine wissenschaftliche Erklärung abgegeben werden soll, weisen die Ergebnisse darauf hin, dass weniger die Polarität einer Ionischen Flüssigkeit, als vielmehr das Vorhandensein von stark koordinierenden Anionen in einer Ionischen Flüssigkeit, wie z. B. Methylsulfat und Ethylsulfat, die Aufnähme an HCl oder HBr beeinflusst. Schwach koordinierende bzw. "nichtkoordinierende" Anionen (S.H. Strauss, Chem. Rev. 1993, 93, 927-942) wie z.B. SO₃CF₃ und besonders PF₆ und BF₄, bei welchen die negative Ladung stark delokalisiert ist, zeigen keinen besonders starken Effekt zur HCl- oder HBr-Absorption. Ionische Flüssigkeiten mit dem SO₃CF₃ -Anion weisen allerdings den verfahrenstechnischen Vorteil auf, das sie gegenüber HCl und anderen Gasgemischbestandteilen sehr stabil sind. Eine etwas geringere Affinität gegenüber HCl wird hier durch hohe Stabilität aufgewogen. Gewünschtenfalls wird das Absorptionsverfahren mittels ionischer Flüssigkeiten, die SO₃CF₃ als Anion aufweisen, zweimal oder mehrmals durchgeführt, bis die gewünschte Abreicherung an HCl erreicht ist. Weitere Anionen, die sich als sehr gut brauchbar erwiesen haben, sind die drei isomeren Tosylat-Anionen (o-Toluolsulfonat, m-Toluolsulfonat und besonders p-Toluolsulfonat). Ionische Flüssigkeiten mit dem Tosylat-Anion können bei Raumtemperatur in flüssiger oder fester Form vorliegen. Ionische Flüssigkeiten mit dem Tosylatanion kann man gewünschtenfalls in Form von Gemischen mit anderen, den Schmelzpunkt erniedrigenden ionischen Flüssigkeiten einsetzen (dies gilt natürlich auch für anderen ionische Flüssigkeiten, die einen höheren Schmelzpunkt als gewünscht haben). Ionische Flüssigkeiten, die ein Anionengemisch von Triflat und Tosylat enthalten oder bevorzugt daraus bestehen, z. B. im Molverhältnis von 0.1:1 bis 10:1, bevorzugt 3:7 bis 7:3, stellen einen guten Kompromiss aus Effektivität zur Abreicherung und Stabilität dar und weisen einen ausreichend niedrigen Schmelzpunkt auf, z. B. im Bereich von 0 bis 60 °C. Aus dem genannten Gemisch bestehend bedeutet, dass keine ionischen Flüssigkeiten mit anderen Anionen als Triflat und Tosylat enthalten.

Bevorzugte ionische Flüssigkeiten sind oftmals solche mit einem E_{T}(30)-Wert von mehr als 20, bevorzugt mehr als 30, insbesondere mehr als 40. Dieser Wert ist ein Maß für die Polarität, siehe Reichardt, Solvent Effects in Organic Chemistry, Weinheim VCH, 1979, XI (Monographs in Modern Chemistry; 3) Seite 241 beschrieben.

Im erfindungsgemäßen Verfahren können ionische Flüssigkeiten eingesetzt werden, die nur eine Verbindung enthalten. Es können auch Gemische von zwei, drei oder mehr verschiedenen ionischen Flüssigkeiten eingesetzt werden. Dadurch kann man z. B. die Trenneigenschaften beeinflussen, beispielsweise die Polarität oder die Affinität zu einer abzutrennenden Verbindung; oder es kann die Viskosität oder die Temperatur beeinflusst werden, bei welcher das Gemisch fest wird. Das letztere wird bei Gemischen von 1-Ethyl-3-methyl-imidazolium-Triflat und 1-Ethyl-3-methyl-imidazolium-Tosylat ausgenutzt.

Der Kontakt zwischen dem zu behandelnden Gasgemisch und der ionischen Flüssigkeit kann nach Verfahren durchgeführt werden, wie sie bei Gas-Flüssig-Operationen üblich sind. Beispielsweise kann man das zu behandelnde Gasgemisch durch die ionische Flüssigkeit hindurchleiten; durch geeignete Düsen, Fritten oder Mischeinrichtungen kann die Kontaktfläche vergrößert werden. Möglich ist z. B. ein Kontakt in einer Blasensäule. Gewünschtenfalls kann die ionische Flüssigkeit auch immobilisiert sein, z. B. auf einem Träger, z. B. einem keramischen Material oder inkorporiert in einem Polymer; diese Ausführungsform ist weniger bevorzugt.

Der Druck ist in einem weiten Bereich variabel, er kann beispielsweise zwischen Umgebungsdruck bis hin zu 10 bar (absolut) oder auch höher liegen. Verfahrenstechnisch am einfachsten ist die Durchführung bei Umgebungsdruck oder einem leichten Überdruck, um das Gasgemisch in die ionische Flüssigkeit zu drücken.

Die Temperatur ist ebenfalls in einem weiten Bereich variabel. Zweckmäßig wählt man die Temperatur derart, dass die Viskosität im gewünschten Bereich liegt. Ein Temperaturbereich, der sich grundsätzlich von der Zersetzungstemperatur bis zur Verfestigungstemperatur, vorzugsweise von 100 °C bis hinunter zur Verfestigungstemperatur der ionischen Flüssigkeit bzw. des Gemisches davon erstreckt, ist möglich. Bevorzugt liegt die Temperatur der ionischen Flüssigkeit beim Kontakt mit dem zu trennenden Gemisch im Bereich zwischen der Verfestigungstemperatur der ionischen Flüssigkeit, z. B. oberhalb von 10 °C, bis hin zu 80 °C. Wie gesagt, ist grundsätzlich ein Bereich von der Verfestigungs- bis zur Zersetzungstemperatur möglich.

Idealerweise wird HCl bzw. HF in der ionischen Flüssigkeit zurückgehalten, und C(O)F₂, PF₅ oder Säurefl uorid passiert die ionische Flüssigkeit. Bei genügender Reinheit können die Gase kondensiert und der jeweiligen Verwendung zugeführt werden. Bei manchen Trennproblemen können weitere Bestandteile im Gasgemisch enthalten sein, die ebenfalls die ionische Flüssigkeit passieren. Bei Gasgemischen von C(O)F₂ und HCl aus der photochemischen Oxidation von CHClF₂ kann z. B. noch Edukt enthalten sein. Die Gasgemischbestandteile, die nicht in der ionischen Flüssigkeit zurückgehalten werden, können, können bereits vor der Durchleitung durch die ionische Flüssigkeit in üblicher Weise, z. B. durch fraktionierte Destillation oder Kondensation, abgetrennt werden. Alternativ kann man sie im zu behandelnden Gasgemisch belassen und nach dem Kontakt mit der ionischen Flüssigkeit abtrennen. Ein Beispiel ist eine fraktionierte Kondensation oder eine Kondensation, an die sich gewünschtenfalls eine Tiefternperaturdestillation anschließen kann.

Die in der ionischen Flüssigkeit zurückgehaltenen Bestandteile können aus dieser physikalisch in einer Rekonditionierung gewonnen werden, z. B. durch Anlegen eines Vakuums, Erwärmung, Durchleiten von Inertgasen oder ähnlichem, gewünschtenfalls auch durch Kombination zweier oder mehrerer solcher physikalischen Maßnahmen wie Temperaturerhöhung und Anlegen eines Vakuums. Durch die Abtrennung der in der ionischen Flüssigkeit zurückgehaltenen Bestandteile des Gasgemisches wird die ionische Flüssigkeit gleichzeitig regeneriert und ist für die erneute Anwendung brauchbar. Die Desorptionstemperatur liegt bevorzugt nicht höher als 100 °C, kann aber bis unterhalb der Zersetzungstemperatur der ionischen Flüssigkeit gehen. Bei Anlegen eines Vakuums ist 1 mbar eine aus technischen Gründen oftmals bevorzugte Grenze. Es spricht aber nichts dagegen, bei noch niedrigerem Druck, z. B. 10⁻³ mbar, zu arbeiten, wie erfolgreich durchgeführte Versuche gezeigt haben. Die Desorption erfolgt sehr schnell (z. B. innerhalb von 1 bis 2 Stunden) auch bei Temperaturen bei oder unterhalb 100 °C, bis hinunter zu bevorzugt 40 °C. Die Entfernung der sorbierten Bestandteile gemäß der EP-A 1 394 109 erfordert dagegen vielstündiges Erhitzen auf 150 °C und höher. Entscheidend für die Desorption durch Druckwechsel ist, dass die Desorption bei einem Druck durchgeführt wird, der niedriger ist als der Druck beim Beladen. Sofern man die Kontaktierung von Gasgemisch und ionischer Flüssigkeit bei Überdruck durchführt, z. B. bei 5 oder mehr bar (abs.) kann die anschließende Desorption bereits bei einem Druck bewirkt werden, der bei oder leicht oberhalb des Umgebungsdruckes liegt, z. B. bei 1 oder 1.5 bar (abs.). Natürlich kann man auch hier bei Unterdruck und gewünschtenfalls erhöhter Temperatur desorbieren und die Rekonditionierung der ionischen Flüssigkeit vervollständigen.

Ein weiterer Gegenstand der Erfindung sind Gemische von ionischen FI üssigkeiten, die das Triflat- und Tosylation enthalten. Bevorzugt sind Gemische, in denen das Molverhältnis der ionischen Flüssigkeiten mit dem Tosylatanion bzw. dem Triflatan ion zwischen 0.1:10 und 10:0.1, vorzugsweise 3:7 und 7:3 beträgt. Tosylat bedeutet o-Tosylat, m-Tosylat, bevorzugt p-Tosylat.

Besonders bevorzugt sind Gemische von ionischen Flüssigkeiten, deren Kation verschieden sind oder bevorzugt gleich sind und zur Klasse der Imidazolium-, Imidazolinium-, Pyrazolium-, Oxatriazolium-, Thiatriazolium-, Pyridinium-, Pyradizinium-, Pyrimidinium- oder Pyrazinium-Verbindungen gehört und insbesondere ausgewählt sind aus 1,3-Dimethyl-imidazolium, 1-Ethyl-3-methyl-imidazolium ("EMIM"), 1-Propyl-3-methylimidazolium und 1-n-Butyl-3-methyl-imidazolium ("BMIM").

In den erfindungsgemäßen Gemischen liegt der Anteil an ionischen Flüssigkeiten mit dem Triflat- und Tosylatanion bevorzugt bei mindestens 75 Gew.-% der Gesamtmenge an ionischer Flüssigkeiten. Der Rest auf 100 Gew.-% wird durch ionische Flüssigkeiten mit anderen Anionen, z. B. dem Ethylsulfat-Anion, gebildet. Bevorzugt liegt der Anteil an ionischen Flüssigkeiten mit dem Triflat- und Tosylatanion bei mindestens 90 Gew.-%, ganz besonders bei 100 Gew.-%. Insbesondere bevorzugte Gemische umfassen EMIM-Triflat und EMIM-Tosylat, oder sie bestehen aus den beiden genannten Komponenten.

Das erfindungsgemäße Verfahren betrifft ein neues Trennproblem, das im Stand der Technik nicht bekannt war. Es unterscheidet sich von bekannten Verfahren dadurch, dass anorganische Bestandteile (HCl, HBr, HF) abgetrennt werden bzw. beteiligt sind, und dass nicht Amine, sondern ionische Flüssigkeiten für die Abtrennung eingesetzt werden. Es hat den Vorteil, dass HCl, HF und HBr auf einfache Weise von anderen hydrolyseempfindlichen Bestandteilen einer Mischung, wie gasförmigen Carbonsäurechloriden und Carbonsäurefluoriden, insbesondere von Phosphorpentafluorid bzw. C(O)F₂, abgetrennt werden können. Besonders vorteilhaft ist die Möglichkeit, HCl aus Gemischen mit C(O)F₂ abzutrennen, die herstellungsbedingt anfallen. Es wird hierbei ein deutlich besserer Trennungsgrad erreicht als z. B. in den Veröffentlichungen US3,253,029 und US 4,092,403, wo Acetonitril als Trennmittel eingesetzt wird. Des Weiteren besitzt die Rekonditionierung einer ionischen Flüssigkeit deutliche Vorteile gegenüber dem Recycling eines organischen Lösungsmittels wie z. B. Acetonitril oder eines Hydrochlorids eines Amins. Man kann auch Gemische behandeln, die aus der Fluorierungsreaktionen von Phosphor-Chlor-Verbindungen mit HF stammen, insbesondere aus der Herstellung von PF₅ aus Phosphor(III)chloriden oder Phosphor(V)chloriden und HFsowie ggf. Cl₂ oder F₂.

Die folgenden Beispiele sollen die Erfindung weiter erläutern, ohne sie in ihrem Umfang einzuschränken.

### Allgemeines

Bei den folgenden Beispielen wurden teilweise Testgemische verwendet, die für die Versuche hergestellt wurden und nur zwei voneinander zu trennende Bestandteile enthielten. Für andere Beispiele wurden Gemische eingesetzt, die aus Photoxidationsversuchen stammen und neben den zu trennenden Bestandteilen C(O)F₂ und HCl weitere Komponenten enthielten, insbesondere die Ausgangsverbindung CHClF₂. Diese Verbindung passiert die ionische Flüssigkeit zusammen mit dem Carbonylfluorid. Die weitere Aufreinigung der resultierenden Gemische kann durch Destillation erfolgen - die weiteren Komponenten werden dabei abgetrennt. Alternativ kann auch zunächst eine Aufreinigung, z. B. unter Destillation des weitere Komponenten enthaltenden Reaktionsgemisches, durchgeführt werden, um die Reaktionskomponenten vorab abzutrennen; das resultierende Gasgemisch enthält dann nur noch Carbonylfluorid und HCl, die dann mittels der ionischen Flüssigkeit getrennt werden.

Tosylat steht bei den Versuchen immer für p-Tosylat.

### Beispiel 1: Abtrennung von HCl aus einem Gemisch mit C(O)F₂

### 1.1. Verwendung von 1-Ethyl-3-methyl-imidazoliumethylsulfat bei Raumtemperatur

Es wurde ein C(O)F₂/HCl-Gasgemisch nach dem Verfahren der eingangs erwähnten noch nicht vorveröffentlichte Europäischen Patentanmeldung 04 00 5421.5 durch Photooxidation von CHF₂Cl hergestellt. Ein Rohgasgemisch der Zusammensetzung 45 % C(O)F₂, 34 % HCl, 6 % O₂ und weiteren Reaktionskomponenten (Edukte u.a.) wurde mit einer Durchflussmenge von 0,8 mol/h unter leicht erhöhtem Druck durch eine Waschflasche mit 115 g (0.49 mol) 1-Ethyl-3-methyl-imidazoliumethylsulfat ("EMIM-ethylsu Ifat") geleitet. Vorteilhaft für den Erhalt von hydrolyseempfindlichen Komponenten (wie z. B. C(O)F₂) eines Gasgemisches ist der Ausschluss bzw. Die Minimierung von Feuchtigkeit in der Apparatur und in der ionischen Flüssigkeit. Dies wurde durch Spülen der Apparatur mit trockenem Stickstoff und Evakuieren unter Erwärmen der ionischen Flüssigkeit erreicht.

Direkt nach Versuchsbeginn wurde hinter der Waschflasche mittels Gaschromatographie folgende Gaszusammensetzung detektiert: 67 % C(O)F₂, 0,0 % HCl, 9 % O₂ und 24 % weitere Reaktionskomponenten. Der prozentuale Anteil der anderen im Gasgemisch enthaltenen Komponenten verringerte sich beim Durchleiten durch die ionische Flüssigkeit nicht.

### 1.2. Verwendung von Acetonitril (Vergleichsbeispiel)

Die Versuchsdurchführung erfolgte wie unter 1.1. beschrieben.

Ein Rohgasgemisch der Zusammensetzung 47 % C(O)F₂, 30 % HCl, 9 % O₂ und weiteren 14 % Reaktionskomponenten wurde mit einem Feed von 0,8 mol/h durch eine Waschflasche mit 83 g (2 mol) Acetonitril geleitet. Direkt nach Versuchsbeginn wurde hinter der Waschflasche mittels Gaschromatographie folgende Gaszusammensetzung detektiert: 55 % C(O)F₂, 12 % HCl, 15 % O₂ und 18 % weitere Reaktionskomponenten.

### 1.3. Rekonditionierung der ionischen Flüssigkeit

Da eine Eigenschaft ionische Flüssigkeiten oder Gemische ionischer Flüssigkeiten ein nicht messbarer Dampfdruck ist, kann zu ihrer Rekonditionierung ein Vakuum von 10 mbar oder niedriger an die beladene Flüssigkeit angelegt werden.

Die mit HCl beladene ionische Flüssigkeit aus Beispiel 1.1. wurde bei einem verringertem Druck von 10 mbar für 1 h gehalten. Ein leichtes Erwärmen z. B. auf 40 °C unter Rühren bei einer bei Raumtemperatur flüssigen ionischen Flüssigkeit beschleunigte die Gasaustreibung. Es wurde solange evakuiert, bis kein Gas mehr aus der ionischen Flüssigkeit entwich.

Wiederholungen des Versuchs 1.3 wurden bei 1 mbar und bei 1⁻³ mbar durchgeführt und führten wieder zur Rekonditionierung der ionischen Flüssigkeit.

Eine Temperaturerhöhung auf z. B. 100 °C sollte erwartungsgemäß ebenfalls eine Desorption unter Rekonditionierung bewirken, bei verringertem Zeitbedarf.

Mittels einfacher Massenbilanz der ionischen Flüssigkeit vor und nach der Gasein leitung lässt sich leicht feststellen, ob die ionische Flüssigkeit vollständig rekonditioniert ist.

### 1.4. Verwendung von 1-Ethyl-3-methyl-imidazoliumethylsulfat bei 0 °C

Wie in Beispiel 1.1. wurde ein Gasgemisch aus der Photooxidation von CHClF₂ eingesetzt, das diesmal 40 % C(O)F₂, 32 % HCl und weitere Reaktionskomponenten enthielt. Das Rohgas wurde mit einer Durchflussmenge von 0.8 mol/h unter leicht erhöhtern Druck durch eine (vorher mit N₂ gespülte) Waschflasche mit 115 g EMIM-ethylsulfat (durch Anlegen eines Vakuums bei etwa 50 °C weitgehend wasserfrei gemacht) geleitet. Nach Beginn des Einleitens wurde mittels Gaschromatographie hinter der Waschflasche folgende Gaszusammensetzung ermittelt: 58 % C(O)F₂, 0 % HCl, 42 % weitere Reaktionskomponenten, die durch Destillation abgetrennt werden können.

### 1.5. Verwendung von 1-Ethyl-3-methyl-imidazoliumtrifluormethansulfonat ("EMIM-triflat") bei Raumtemperatur

Eingesetzt wurde ein wiederum durch Photooxidation von CHClF₂ erhaltenes Gasgemisch mit 40 % C(O)F₂, 32 % HCl und weiteren Reaktionskomponenten. Die Durchleitung erfolgte mit einer Durchflussmenge von 0.8 mol/h unter leicht erhöhtem Druck durch eine (wieder mit N₂ gespülte) Waschflasche mit 100 g (0.38 mol) EMIM-triflat (durch Anlegen eines Vakuums bei etwa 50 °C weitgehend wasserfrei gemacht). Nach Versuchsbeginn wurde hinter der Waschflasche mittels Gaschromatographie folgende Gaszusammensetzung detektiert: 53 % C(O)F₂, 5 % HCl, 42 % weitere Reaktionskomponenten.

Der HCl-Gehalt kann durch eine weitere Kontaktierung mit EMIM-triflat weiter reduziert werden.

### 1.6. Verwendung von 1-Ethyl-3-methyl-imidazoliumtoluolsulfonat ("EMIM-tosylat") bei 60 °C

Das in 1.5. verwendete Reaktionsgemisch wurde, nach Trocknung der Apparatur mittels N₂ und Vakuumbeaufschlagung der ionischen Flüssigkeit bei 100 °C, durch 100 g (0.35 mol) EMIM-tosylat geleitet. Nach Versuchsbeginn wurde hinter der Waschflasche mittels Gaschromatographie folgende Gaszusammensetzung detektiert: 56 % C(O)F₂, 0 % HCl, 44 % weitere Reaktionskomponenten.

### 1.7. Verwendung eines Gemischtes von 1-Ethyl-3-methyl-imidazoliumtrifluormethansulfonat und 1-Ethyl-3-methyl-imidazoliumtoluolsulfonat bei 0 °C

EMIM-triflat und EMIM-tosylat wurden im Molverhältnis 1:1 eingesetzt und wie oben beschrieben etwaiges Wasser entfernt. Diesmal wurde ein Rohgasgemisch eingesetzt, das 41 % C(O)F₂, 32 % HCl und weitere Reaktionskomponenten aus der Photooxidation von CHClF₂ enthielt. Die Durchflussmenge betrug wieder 0.8 mol/h, die Gesamtmenge an ionischer Flüssigkeit betrug 100 g. Nach Versuchsbeginn wurde hinter der Waschflasche mittels Gaschromatographie folgende Gaszusammensetzung detektiert: 63 % C(O)F₂, 2 % HCl, 35 % weitere Reaktionskomponenten.

Auch bei diesem Versuch kann bei erneutem Kontaktieren mit Gemisch der ionischen Flüssigkeiten der Gehalt an HCl weiter abgesenkt werden.

### Beispiel 2: Abtrennung von HCl aus PF₅ mittels EMIM-triflat

Durch Kondensation entsprechender Mengen an PF₅ und HCl wurde ein Testgemisch mit 56 % PF₅ und 44 % HCl erzeugt. Diese Mischung wurde bei Raumtemperatur mit einer Durchflussmenge von 0.8 mol/h für 15 min durch eine Waschflasche mit 100 g EMIM-triflat geleitet. Dabei belud sich die ionische Flüssigkeit mit HCl. Es wurde dann eine Desorption vorgenommen, indem bei 100 °C für 30 min ein Vakuum von 1 mbar angelegt wurde.

Da PF₅ gaschromatographisch schwer detektiert werden kann (es reagiert mit dem SiO₂ der Säule), wurde folgendermaßen vorgegangen: Der Abgasstrom aus der Desorption wurde durch eine Gaswaschflasche mit 100 g 0.1 n-NaOH-Lösung (Titrinorm, VWR) geleitet. Die Ionenchromatographie der erhaltenen sauren Waschlösung ergab ein Verhältnis von Chlorid zu Fluorid von 95:5. Zum Vergleich wurde das Ausgangsgemisch (44 % HCl, Rest auf 100 % PF₅) durch 100 g der 0.1n-NaOH-Lösung geleitet. Ionenchromatographie ergab ein Verhältnis von Chlorid zu Fluorid von 60 zu 40. Dieser Vergleich belegt, dass in der ionischen Flüssigkeit HCl stark bevorzugt gegenüber PF₅ absorbiert wird.

Aus der Massenbilanz der ionischen Flüssigkeit vor und nach der Desorption wurde auf eine vollständige Desorption geschlossen.

### Beispiel 3: Abtrennung von Fluorwasserstoffsäure (HF) und Chlorwasserstoffsäure (HCl) aus Trifluoracetylfluorid (TFAF) mittels EMIM-trifluormethansulfonsäure

Trifluoracetylchlorid (TFAC) wurde mit Fluorwasserstoffsäure (HF) im Autoklaven über Nacht bei 50 °C gerührt. Das entstehende Gemisch aus TFAF und HCl, sowie nicht umgesetztes TFAC und HF wurde durch 100g EMIM-triflat geleitet. Die Zusammensetzung der Gasmischung vor der Durchleitung durch die ionische Flüssigke it wurde gaschromatisch ermittelt (56 % TFAF, 34 % HCl, 7% TFAC). Vorhandenes HF im Gasgemisch wurde qualitativ durch angeätzte Oberflächen von Glas vor der Durchströmung der ionischen Flüssigkeit festgestellt.

Nach dem Passieren der ionischen Flüssigkeit wurde folgende Zusammensetzung des Gasgemisches gaschromatographisch bestimmt: 86 % TFAF, 2 % HCl, 11 % TFAC. Eine Analyse der beladenen ionischen Flüssigkeit auf Halogenidionen ergab neben den zu erwartenden Chloridanionen auch Fluoridanionen. Diese Ionen waren in der unbeladenen ionischen Flüssigkeit nicht enthalten, des Weiteren liegt der gefundene Fluorid-Wert deutlich höher als er z. B. durch Hydrolyse von TFAF durch Spuren an Feuchtigkeit in der ionischen Flüssigkeit erhalten werden kann.

Es wurde auch festgestellt, dass das die ionische Flüssigkeit verlassende Gasgemisch Glasoberflächen nicht mehr ätzte.

## Patentansprüche

1. Verfahren zur Abtrennung von HCl, HF oder HBr aus Gasgemischen, die HCl, HF oder HBr und ein oder mehrere andere gasförmige Bestandteile enth alten, unter Kontaktieren dieser Gasgemische mit einer oder mehreren ionischen Flüssigkeiten, die HCl, HF oder HBr oder mindestens einen der anderen Bestandteile des Gasgemisches unterschiedlich stark sorbieren.

2. Verfahren nach Anspruch 1 zur Gewinnung von C(O)F₂, das an HCl abgereichert ist, aus Gemischen, die C(O)F₂ und HCl enthalten, unter Kontaktie ren dieser Gemische mit einer oder mehreren ionischen Flüssigkeiten, die C(O)F₂ und HCl unterschiedlich stark sorbieren.

3. Verfahren nach Anspruch 1 zur Gewinnung von PF₅, das an HCl abgereichert ist, aus Gemischen, die PF₅ und HCl enthalten, unter Kontaktieren dieser Gemische mit einer oder mehreren ionischen Flüssigkeiten, die PF₅ und HCl unterschiedlich stark sorbieren.

4. Verfahren nach Anspruch 1 zur Gewinnung von Carbonsäurefl uorid, das an HCl abgereichert ist, aus Gemischen, die Carbonsäurefluorid und HCl enthalten, unter Kontaktieren dieser Gemische mit einer oder mehreren ionischen Flüssigkeiten , die Carbonsäurefluorid und HCl unterschiedlich stark sorbieren, wobei das Gemisch gegebenenfalls HF enthalten kann, das ebenfalls abgetrennt wird.

5. Verfahren nach Anspruch 1, **dadurch gekennzeichnet, dass** das Carbonsäurefluorid CH₃C(O)F, CHF₂C(O)F, CF₃C(O)F oder C₂F₅C(O)F ist.

6. Verfahren nach einem der Ansprüche 2 bis 5, **dadurch gekennzeichnet, dass** man eine oder mehrere ionische Flüssigkeiten verwendet, die Carbonsä urefluorid, C(O)F₂ bzw. Phosphorpentafluorid stärker sorbieren als HCl oder HF, oder dass man bevorzugt eine oder mehrere ionische Flüssigkeiten verwendet, die HCl oder HF stärker sorbieren als Carbonsäurefluorid, C(O)F₂ bzw. Phosphorpentafluorid.

7. Verfahren nach Anspruch 1, **dadurch gekennzeichnet, dass** man eine ionische Flüssigkeit verwendet, die im Kation Stickstoff enthält.

8. Verfahren nach Anspruch 1, **dadurch gekennzeichnet, dass** man eine ionische Flüssigkeit verwendet, die ein Anion aufweist, welches einen E_{T}(30)-Wert von mehr als 20 aufweist und das zur Koordination befähigt ist.

9. Verfahren nach Anspruch 1, **dadurch gekennzeichnet, dass** das Anion der ionischen Flüssigkeit ein Sulfonat, Sulfonamid oder ein Esteranion einer mehrbasigen Säure ist.

10. Verfahren nach Anspruch 6, **dadurch gekennzeichnet, dass** das Anion ein Monoalkylsulfat-Anion, Monoarylsulfat-Anion, ein verestertes Phosphat- oder Phosphonat-Anion ist, wobei die Alkyl- Aryl- oder Estergruppen gewünschtenfalls durch 1 oder mehrere Halogenatome, vorzugsweise Fluoratome, substituiert sein können.

11. Verfahren nach Anspruch 10, **dadurch gekennzeichnet, dass** das Anion ei n Mono-C1-C12-Alkylsulfat, vorzugsweise ein C1-C4-Monoalkylsulfat-Anion ist.

12. Verfahren nach Anspruch 10, **dadurch gekennzeichnet, dass** die ionische Flüssigkeit das Anion p-Tosylat, m-Tosylat, o-Tosylat und/oder Triflat enthält.

13. Verfahren nach Anspruch 12, **dadurch gekennzeichnet, dass** die ionische Flüssigkeit ein Gemisch aus mindestens zwei ionischen Flüssigkeiten mit dem p-Tosylat-Anion, dem o-Tosylat-Anion und/oder dem m-Tosylat-Anion ist oder ein Gemisch aus einer oder mehreren ionischen Flüssigkeiten mit dem p-Tosylat-Anion, dem o-Tosylat-Anion oder dem m-Tosylat-Anion, und mindestens einer weiteren ionischen Flüssigkeit mit einem anderen Anion, vorzugsweise dem Triflat-Anion.

14. Verfahren nach Anspruch 7, **dadurch gekennzeichnet, dass** das Kation zur Klasse der Imidazolium-, Imidazolinium-, Pyrazolium-, Oxatriazolium-, Thiatriazolium-, Pyridinium-, Pyradizinium-, Pyrimidinium- oder Pyrazinium-Verbindungen gehört.

15. Verfahren nach Anspruch 14, **dadurch gekennzeichnet, dass** das Kation ein Imidazoliumkation ist, das durch ein, zwei oder drei Substituenten mit je 1-24 Kohlenstoffatomen substituiert ist, wobei die Substituenten ihrerseits substituiert sein können, beispielsweise durch Arylgruppen wie Phenyl.

16. Verfahren nach Anspruch 15, **dadurch gekennzeichnet, dass** das Kation 1,3-Dimethyl-imidazolium, 1-Ethyl-3-methyl-imidazolium, 1-Propyl-3-methyl-imidazolium oder 1-n-Butyl-3-methyl-imidazolium ist.

17. Verfahren nach Anspruch 16, **dadurch gekennzeichnet, dass** man als ionische Flüssigkeit 1-Ethyl-3-methyl-imidazolium-ethylsulfat, 1-Ethyl-3-methyl-imidazolium-tosylat, 1-Ethyl-3-methyl-imidazolium-triflat oder deren Gemische verwendet, wobei "tosylat" für o-tosylat, m-tosylat oder p-tosylat steht und bevorzugt p-tosylat bedeutet.

18. Verfahren nach Anspruch 1, **dadurch gekennzeichnet, das** das zu behandelnde Gemisch, vorzugsweise gasförmig, durch die ionische Flüssigkeit hindurchgeleitet wird.

19. Verfahren nach Anspruch 11, **dadurch gekennzeichnet, dass** die ionische Flüssigkeit immobilisiert vorliegt.

20. Verfahren nach Anspruch 1, **dadurch gekennzeichnet, dass** man den Kontakt zwischen ionischer Flüssigkeit und dem zu trennenden Gemisch bei einer Temperatur im Bereich von 10 bis 80 °C und einem Druck von 1 bis 10 bar (abs) durchführt.

21. Verfahren nach Anspruch 1, **dadurch gekennzeichnet, dass** die in der ionischen Flüssigkeit sorbierten Bestandteile desorbiert werden, um die ionische Flüssigkeit zurückzugewinnen und/oder um die sorbierten Bestandteile zu gewinnen.

22. Verfahren nach Anspruch 21, **dadurch gekennzeichnet, dass** man die Desorption durch Druckerniedrigung, Temperaturerhöhung und/oder Durchleiten eines inerten Gases bewirkt.

23. Verfahren nach Anspruch 2, **dadurch gekennzeichnet, dass** man Gemische behandelt, die aus der photochemischen Oxidation von CHClF₂ zum Zwecke der Herstellung von C(O)F₂ stammen, oder aus Fluorierungsreaktionen von Phosphor-Chlor-Verbindungen mit HF, insbesondere aus der Herstellung von PF₅ aus Phosphor(III)chloriden oder Phosphor(V)chloriden und HF sowie ggf. Cl₂ oder F₂, oder aus Verfahren zur Herstellung von bei 25°C und 1 bar (abs.) gasförmigen Carbonsäurefluoriden, insbesondere aus Verfahren, die die photochemische Oxidation von Chlorfluoralkanen oder die Fluorierung von Carbonsäurechloriden mit HF umfassen, wobei Carbonsäurefluorid bevorzugt für CH₃C(O)F, CHF₂C(O)F, CF₃C(O)F oder C₂F₅C(O)F steht.

24. Verfahren nach Anspruch 1, **dadurch gekennzeichnet, dass** es batchweise oder bevorzugt kontinuierlich durchgeführt wird.

25. Gemische von ionischen Flüssigkeiten, die das Triflat- und Tosylation enthalten.

26. Gemische nach Anspruch 25, wobei das Molverhältnis der ionischen Flüssigkeiten mit dem Tosylatanion bzw. dem Triflatanion zwischen 3:7 und 7:3 beträgt.

27. Gemische nach Anspruch 25 oder 26, **dadurch gekennzeichnet, dass** das Kation der ionischen Flüssigkeiten verschieden oder bevorzugt gleich ist und zur Klasse der Imidazolium-, Imidazolinium-, Pyrazolium-, Oxatriazolium-, Thiatriazolium-, Pyridinium-, Pyradizinium-, Pyrimidinium- oder Pyrazinium-Verbindungen gehört.

28. Gemische nach Anspruch 27, **dadurch gekennzeichnet, dass** die Kationen der ionischen Flüssigkeiten verschieden oder bevorzugt gleich sind und ausgewählt sind aus 1,3-Dimethyl-imidazolium, 1-Ethyl-3-methyl-imidazolium, 1-Propyl-3-methyl-imidazolium und 1-n-Butyl-3-methyl-imidazolium.

29. Gemische nach Anspruch 25, **dadurch gekennzeichnet, dass** der Anteil der ionischen Flüssigkeiten mit dem Triflat- und Tosylatanion mindestens 75 Gew.-% der Gesamtmenge an ionischer Flüssigkeiten, bevorzugt 100 Gew.-%, beträgt.

## Claims

1. Process for separating HCl, HF or HBr from gas mixtures containing HCl, HF or HBr and one or more other gaseous constituents, wherein these gas mixtures are contacted with one or more ionic liquids which sorb HCl, HF or HBr or at least one of the other constituents of the gas mixture to a differing degree.

2. Process according to Claim 1 for isolating C(O)F₂ which is depleted in HCl from mixtures containing C(O)F₂ and HCl, wherein these mixtures are contacted with one or more ionic liquids which sorb C(O)F₂ and HCl to differing degrees.

3. Process according to Claim 1 for isolating PF₅ which is depleted in HCl from mixtures containing PF₅ and HCl, wherein these mixtures are contacted with one or more ionic liquids which sorb PF₅ and HCl to differing degrees.

4. Process according to Claim 1 for isolating carboxylic acid fluoride which is depleted in HCl from mixtures containing carboxylic acid fluoride and HCl, wherein these mixtures are contacted with one or more ionic liquids which sorb carboxylic acid fluoride and HCl to differing degrees, with the mixture being able to contain HF which is likewise separated off.

5. Process according to Claim 1, **characterized in that** the carboxylic acid fluoride is CH₃C(O)F, CHF₂C(O)F, CF₃C(O)F or C₂F₅C(O)F.

6. Process according to any of Claims 2 to 5, **characterized in that** one or more ionic liquids which sorb carboxylic acid fluoride, C(O)F₂ or phosphorus pentafluoride more strongly than HCl or HF are used or **in that** one or more ionic liquids which sorb HCl or HF more strongly than carboxylic acid fluoride, C(O)F₂ or phosphorus pentafluoride are preferably used.

7. Process according to Claim 1, **characterized in that** an ionic liquid containing nitrogen in the cation is used.

8. Process according to Claim 1, **characterized in that** an ionic liquid which has an anion which has an E_{T}(30) value of more than 20 and is capable of coordination is used.

9. Process according to Claim 1, **characterized in that** the anion of the ionic liquid is a sulphonate, sulphonamide or an ester anion of a polybasic acid.

10. Process according to Claim 6, **characterized in that** the anion is a monoalkylsulphate anion, monoarylsulphate anion, an esterified phosphate or phosphonate anion, where the alkyl, aryl or ester groups may, if desired, be substituted by 1 or more halogen atoms, preferably fluorine atoms.

11. Process according to Claim 10, **characterized in that** the anion is a mono-C1-C12-alkylsulphate, preferably a C1-C4-monoalkylsulphate anion.

12. Process according to Claim 10, **characterized in that** the ionic liquid contains the anion p-tosylate, m-tosylate, o-tosylate and/or triflate.

13. Process according to Claim 12, **characterized in that** the ionic liquid is a mixture of at least two ionic liquids having the p-tosylate anion, the o-tosylate anion and/or the m-tosylate anion or a mixture of one or more ionic liquids having the p-tosylate anion, the o-tosylate anion or the m-tosylate anion and at least one further ionic liquid having another anion, preferably the triflate anion.

14. Process according to Claim 7, **characterized in that** the cation belongs to the class of imidazolium, imidazolinium, pyrazolium, oxatriazolium, thiatriazolium, pyridinium, pyradizinium, pyrimidinium and pyrazinium compounds.

15. Process according to Claim 14, **characterized in that** the cation is an imidazolium cation which is substituted by one, two or three substituents each having 1-24 carbon atoms, where the substituents may in turn be substituted, for example by aryl groups such as phenyl.

16. Process according to Claim 15, **characterized in that** the cation is 1,3-dimethylimidazolium, 1-ethyl-3-methylimidazolium, 1-propyl-3-methylimidazolium or 1-n-butyl-3-methylimidazolium.

17. Process according to Claim 16, **characterized in that** 1-ethyl-3-methylimidazolium ethylsulphate, 1-ethyl-3-methylimidazolium tosylate, 1-ethyl-3-methylimidazolium triflate or a mixture thereof, where "tosylate" is o-tosylate, m-tosylate or p-tosylate and preferably p-tosylate, is used as ionic liquid.

18. Process according to Claim 1, **characterized in that** the mixture to be treated is passed, preferably in gaseous form, through the ionic liquid.

19. Process according to Claim 11, **characterized in that** the ionic liquid is present in immobilised form.

20. Process according to Claim 1, **characterized in that** the contacting of the ionic liquid and the mixture to be separated is carried out at a temperature in the range from 10 to 80°C and a pressure of from 1 to 10 bar (abs).

21. Process according to Claim 1, **characterized in that** the constituents sorbed in the ionic liquid are desorbed in order to recover the ionic liquid and/or to isolate the sorbed constituents.

22. Process according to Claim 21, **characterized in that** the desorption is effected by reducing the pressure, increasing the temperature and/or passing an inert gas through the ionic liquid.

23. Process according to Claim 2, **characterized in that** mixtures which originate from the photochemical oxidation of CHClF₂ for the purpose of preparing C(O)F₂ or from fluorination reactions of phosphorus-chlorine compounds with HF, in particular from the preparation of PF₅ from phosphorus(III) chlorides or phosphorus(V) chlorides and HF and also, if appropriate, Cl₂ or F₂, or from processes for preparing carboxylic acid fluorides which are gaseous at 25°C and 1 bar (abs.), in particular for processes which comprise the photochemical oxidation of chlorofluoralkanes or the fluorination of carboxylic acid chlorides by means of HF, where the carboxylic acid fluoride is preferably CH₃C(O)F, CHF₂C(O)F, CF₃C(O)F or C₂F₅C(O)F, are treated.

24. Process according to Claim 1, **characterized in that** it is carried out batchwise or preferably continuously.

25. Mixtures of ionic liquids which contain the triflate ion and tosylate ion.

26. Mixtures according to Claim 25, wherein the molar ratio of the ionic liquids having the tosylate anion and the triflate anion is from 3:7 to 7:3.

27. Mixtures according to Claim 25 or 26, **characterized in that** the cations of the ionic liquids are different or preferably identical and belong to the class of imidazolium, imidazolinium, pyrazolium, oxatriazolium, thiatriazolium, pyridinium, pyradizinium, pyrimidinium and pyrazinium compounds.

28. Mixtures according to Claim 27, **characterized in that** the cations of the ionic liquids are different or preferably identical and are selected from among 1,3-dimethylimidazolium, 1-ethyl-3-methylimidazolium, 1-propyl-3-methylimidazolium and 1-n-butyl-3-methylimidazolium.

29. Mixtures according to Claim 25, **characterized in that** the proportion of the ionic liquids having the triflate and tosylate anions is at least 75 % by weight of the total amount of ionic liquids, preferably 100 % by weight.

## Revendications

1. Procédé de séparation d'HCl, HF ou HBr de mélanges gazeux qui contiennent HCl, HF ou HBr et un ou plusieurs autres constituants gazeux, par mise en contact de ces mélanges gazeux avec un ou plusieurs liquides ioniques qui absorbent HCl, HF ou HBr ou au moins un des autres constituants du mélange gazeux avec différentes forces.

2. Procédé selon la revendication 1, pour la récupération de C(O)F₂ appauvri en HCl à partir de mélanges qui contiennent C(O)F₂ et HCl, par mise en contact de ces mélanges avec un ou plusieurs liquides ioniques qui absorbent C(O)F₂ et HCl avec différentes forces.

3. Procédé selon la revendication 1, pour la récupération de PF₅ appauvri en HCl à partir de mélanges qui contiennent PF₅ et HCl, par mise en contact de ces mélanges avec un ou plusieurs liquides ioniques qui absorbent PF₅ et HCl avec différentes forces.

4. Procédé selon la revendication 1, pour la récupération d'un fluorure d'acide carboxylique appauvri en HCl à partir de mélanges qui contiennent le fluorure d'acide carboxylique et HCl, par mise en contact de ces mélanges avec un ou plusieurs liquides ioniques qui absorbent le fluorure d'acide carboxylique et HCl avec différentes forces, le mélange pouvant éventuellement contenir HF, qui est également séparé.

5. Procédé selon la revendication 1, **caractérisé en ce que** le fluorure d'acide carboxylique est CH₃C(O)F, CHF₂C(O)F, CF₃C(O)F ou C₂F₅C(O)F.

6. Procédé selon l'une quelconque des revendications 2 à 5, **caractérisé en ce qu'**on utilise un ou plusieurs liquides ioniques qui absorbent le fluorure d'acide carboxylique, C(O)F₂ ou le pentafluorure de phosphore plus fortement qu'HCl ou HF, ou **en ce qu'**on utilise de préférence un ou plusieurs liquides ioniques qui absorbent HCl ou HF plus fortement que le fluorure d'acide carboxylique, C(O)F₂ ou le pentafluorure de phosphore.

7. Procédé selon la revendication 1, **caractérisé en ce qu'**on utilise un liquide ionique qui contient de l'azote dans le cation.

8. Procédé selon la revendication 1, **caractérisé en ce qu'**on utilise un liquide ionique qui comporte un anion qui présente une valeur ET(30) supérieure à 20 et qui est apte à une coordination.

9. Procédé selon la revendication 1, **caractérisé en ce que** l'anion du liquide ionique est un sulfonate, un sulfonamide ou un anion d'un ester d'un polyacide.

10. Procédé selon la revendication 6, **caractérisé en ce que** l'anion est un anion monoalkylsulfate, un anion monoarylsulfate, un anion phosphonate ou phosphate estérifié, les groupes alkyle, aryle ou ester pouvant si on le souhaite être substitués par 1 ou plusieurs atomes d'halogène, de préférence atomes de fluor.

11. Procédé selon la revendication 10, **caractérisé en ce que** l'anion est un monoalkylsulfate en C1-C12, de préférence un anion monoalkylsulfate en C1-C4.

12. Procédé selon la revendication 10, **caractérisé en ce que** le liquide ionique contient l'anion p-tosylate, m-tosylate, o-tosylate et/ou triflate.

13. Procédé selon la revendication 12, **caractérisé en ce que** le liquide ionique est un mélange d'au moins deux liquides ioniques contenant l'anion p-tosylate, l'anion o-tosylate et/ou l'anion m-tosylate ou un mélange d'un ou de plusieurs liquides ioniques contenant l'anion p-tosylate, l'anion o-tosylate ou l'anion m-tosylate, et d'au moins un liquide ionique supplémentaire contenant un autre anion, de préférence l'anion triflate.

14. Procédé selon la revendication 7, **caractérisé en ce que** le cation appartient à la classe des composés imidazolium, imidazolinium, pyrazolium, oxatriazolium, thiatriazolium, pyridinium, pyradizinium, pyrimidinium ou pyrazinium.

15. Procédé selon la revendication 14, **caractérisé en ce que** le cation est un cation imidazolium qui est substitué par un, deux ou trois substituants contenant chacun 1 à 24 atomes de carbone, les substituants pouvant eux-mêmes être substitués, par exemple par des groupes aryles tels que phényle.

16. Procédé selon la revendication 15, **caractérisé en ce que** le cation est le 1,3-diméthyl-imidazolium, le 1-éthyl-3-méthyl-imidazolium, le 1-propyl-3-méthyl-imidazolium ou le 1-n-butyl-3-méthyl-imidazolium.

17. Procédé selon la revendication 16, **caractérisé en ce que** le 1-éthyl-3-méthyl-imidazolium-éthylsulfate, le 1-éthyl-3-méthyl-imidazolium-tosylate, le 1-éthyl-3-méthyl-imidazolium-triflate ou leurs mélanges sont utilisés en tant que liquide ionique, « tosylate » signifiant o-tosylate, m-tosylate ou p-tosylate et de préférence p-tosylate.

18. Procédé selon la revendication 1, **caractérisé en ce que** le mélange à traiter, de préférence gazeux, est passé au travers du liquide ionique.

19. Procédé selon la revendication 11, **caractérisé en ce que** le liquide ionique est immobilisé.

20. Procédé selon la revendication 1, **caractérisé en ce que** le contact entre le liquide ionique et le mélange à séparer est réalisé à une température dans la plage allant de 10 à 80 °C et à une pression de 1 à 10 bars (abs.).

21. Procédé selon la revendication 1, **caractérisé en ce que** les constituants absorbés dans le liquide ionique sont désorbés afin de récupérer le liquide ionique et/ou afin de récupérer les constituants absorbés.

22. Procédé selon la revendication 21, **caractérisé en ce que** la désorption est effectuée par diminution de la pression, augmentation de la température et/ou passage d'un gaz inerte.

23. Procédé selon la revendication 2, **caractérisé en ce que** les mélanges traités sont issus de l'oxydation photochimique de CHClF₂ pour la fabrication de C(O)F₂, ou de réactions de fluoration de composés de phosphore-chlore avec HF, notamment de la fabrication de PF₅ à partir de chlorures de phosphore (III) ou de chlorures de phosphore (V) et HF, ainsi qu'éventuellement Cl₂ ou F₂, ou de procédés de fabrication de fluorures d'acides carboxyliques gazeux à 25 °C et 1 bar (abs.), notamment de procédés qui comprennent l'oxydation photochimique d'alcanes chlorofluorés ou la fluoration de chlorures d'acides carboxyliques avec HF, le fluorure d'acide carboxylique étant de préférence CH₃C(O)F, CHF₂C(O)F, CF₃C(O)F ou C₂F₅C(O)F.

24. Procédé selon la revendication 1, **caractérisé en ce qu'**il est réalisé de manière discontinue ou de préférence de manière continue.

25. Mélanges de liquides ioniques, contenant l'ion triflate et tosylate.

26. Mélanges selon la revendication 25, dans lesquels le rapport molaire entre les liquides ioniques qui contiennent l'anion tosylate et qui contiennent l'anion triflate est compris entre 3:7 et 7:3.

27. Mélanges selon la revendication 25 ou 26, **caractérisés en ce que** le cation des liquides ioniques est différent ou de préférence identique et appartient à la classe des composés imidazolium, imidazolinium, pyrazolium, oxatriazolium, thiatriazolium, pyridinium, pyradizinium, pyrimidinium ou pyrazinium.

28. Mélanges selon la revendication 27, **caractérisés en ce que** les cations des liquides ioniques sont différents ou de préférence identiques et sont choisis parmi le 1,3-diméthyl-imidazolium, le 1-éthyl-3-méthyl-imidazolium, le 1-propyl-3-méthyl-imidazolium et le 1-n-butyl-3-méthyl-imidazolium.

29. Mélanges selon la revendication 25, **caractérisés en ce que** la proportion des liquides ioniques contenant l'anion triflate et tosylate est d'au moins 75 % en poids de la totalité des liquides ioniques, de préférence de 100 % en poids.
